# EUROPEAN PATENT SPECIFICATION

(11) **EP 1 865 922 B1**
(45) Date of publication and mention of the grant of the patent: **28.10.2009**
(21) Application number: 06709938.2
(22) Date of filing: 01.03.2006
(51) Int. Cl.: A61K 9/08, A61F 6/04

(54) **PERSONAL LUBRICANT CONTAINING POLYETHYLENE OXIDE**
KÖRPERGLEITMITTEL MIT POLYETHYLENOXID
LUBRIFIANT PERSONNEL CONTENANT DU POLYETHYLENE OXIDE

(30) Priority: 04.03.2005 GB 0504547
(43) Date of publication of application: 19.12.2007
(73) Proprietor: Delsol Products Limited, London W1J 7LU (GB)
(72) Inventor: POTTER, William, D., Babraham Cambridge CB22 3AG (GB)
(74) Representative: Harrison, Ivor Stanley
(86) International application number: PCT/GB2006/000714
(87) International publication number: WO 2006/092585

(56) References cited:
- WO-A-92/09256
- US-A- 5 137 032
- US-A- 5 208 031
- US-A1- 2005 076 917
- RIP N ROLL: "KY Jelly Lubricant"[Online] XP002390402 Retrieved from the Internet: URL:http://www.ripnroll.com/KY-Jelly-lubri cant.htm>

## Description

This invention relates to personal lubricants and to condoms having a surface coating comprising a personal lubricant.

The majority of condoms currently available are pre-lubricated. The lubricant facilitates insertion of the penis into the vagina and improves comfort during intercourse. The lubricant may also contribute to a reduction in the risk of failure of the condom by lowering the friction between the condom and the vaginal wall. The most commonly used lubricant is a silicone fluid, poly(dimethyl siloxane) having a viscosity of about 350 cSt, but other fluids, for example glycols, are also used.

The lubricant serves a number of functions. As well as providing lubrication when the condom is used it also, by migrating along the length of the rolled up condom when packaged, facilitates unrolling the condom as well as preventing the surfaces of the rolled condom sticking together as the product ages in storage. In order to achieve adequate migration of the lubricant along the length of the rolled condom, the lubricant must have good wetting properties for the material of the condom and must not have too high a viscosity. There is therefore a compromise between selecting a lubricant with a high viscosity, which tends to improve lubrication, and one with a low viscosity which will migrate more effectively into the rolls of the packaged condom.

The amount of lubricant which can be incorporated into the pack with the condom is usually limited to approximately 0.5g. Attempts to use significantly larger quantities of lubricant can result in difficulties in sealing the pack and an increase in the incidence of pack leakage during distribution and storage. The limited volume of lubricant that can be used, taken together with the restrictions placed on the maximum viscosity that is acceptable for adequate migration of the lubricant, result in lubrication that is sub-optimal for maximum comfort and condom safety.

In an attempt to overcome the disadvantages associated with the use of silicone fluids as condom lubricants, some condoms carry a coating of a water-based lubricant. Water-based Water-based lubricants may offer some improvement in lubrication but they are still constrained by the general considerations relating to lubricant volume and maximum viscosity as described above. Water based lubricants also suffer additional disadvantages, such as causing the latex from which the condom is formed to hydrate and become white and opaque, not only detracting from the aesthetic appearance of the condom but also causing a loss of strength with some latex formulations, and causing the condom to feel cold to the touch and thus having limited acceptability in many parts of the world.

US patent no. 6,196,227 describes an improved condom lubricant which addresses some of the problems described above. The lubricant is water based and contains a "sliminess" agent, an agent to promote penetration within the rolls of the condom when in the packaged condition, and a humectant. Although this formulation overcomes some of the disadvantages described above, its viscosity, and therefore its lubricating properties, are still constrained by the need to achieve an acceptable level of migration into the rolled up condom.

Many personal lubricants which are sold separately can also be used with a condom. These lubricants are normally water based and are applied to the outside of the condom immediately before vaginal penetration or into the vagina itself. These lubricants can be formulated for optimum lubricating properties but, because of their high viscosity and lack of flow on a rolled condom and the fact that they hydrate the latex film, they are unsuitable for use on pre-lubricated condoms. Some water based personal lubricants contain poly(ethylene oxide), which produces lubricants with high viscosities and a very slippery feel. While these lubricants have excellent lubricating properties they are, however, for the reasons discussed above, unsuitable for use on pre-lubricated condoms.

WO-A-92/09256 describes a water-based lubricant containing a polyethylene oxide as a thickening agent, in combination with a humectant polyol exhibiting high lubricity. US-A-5208031 provides a sexual lubricant containing, in a water-based composition, zinc salts as anti-viral agents, thickening agents such as hydroxyethyl cellulose and lubricating agents such as glycerin or polyethylene glycol. US-A-5137032 describes a condom the external surface of which carries a coating of a weekly adhesive bonding agent to restrict movement of the distal end of the condom within the vagina during intercourse.

In one aspect, the present invention provides a personal lubricant composition comprising a mixture of a latent lubricant additive and a carrier lubricant, in which the latent lubricant additive is insoluble in the carrier lubricant, the latent lubricant additive being in particulate or powder form and water-soluble, and comprising a poly (ethylene oxide) polymer, characterised in that the carrier lubricant comprises one or more polyhydric alcohols whereby the latent lubricant is capable of activation on contact with body fluids to realise lubricant properties.

By "lubricant properties" is meant, on activation, smoothness or slipperiness (lubricity) and stringiness or pituity, the latter implying appearance and behaviour similar to natural mucus. Preferably, the body fluids which will activate the latent lubricant additive are vaginal secretions but saliva may also be effective.

In another aspect, the invention provides a condom having applied thereto a lubricant composition as hereinbefore described.

According to the present invention, the latent lubricant additive is a solid in particulate or powder form and is water-soluble. The additive may comprise a polymer such as a poly(ethylene oxide). Such polymers may have molecular weights ranging from approximately 100,000 (viscosity-average) up to approximately 12,000,000 but we have found that, for use in compositions according to the invention, the molecular weight should preferably be not less than approximately 400,000 for the resulting activated lubricant to have adequate lubricity and not less than 4,000,000 and up to 6,000,000 for the lubricant also to exhibit a pituity similar to that of natural mucus. The polymer is preferably finely-divided and dispersed in the carrier lubricant as a stable suspension. For example, the polymer may be micronised using an air microniser to a particle size where 95% is below 50 micron with a weighted mean particle size of 29 micron.

The carrier lubricant is also water-soluble and, although not having solvating power for the latent lubricant additive, will nevertheless be miscible with the activated additive when in contact with body fluids, especially vaginal secretions during penetration and intercourse, resulting in a homogeneous lubricant without the formation of localised lumps or gels. The carrier lubricant comprises one or more polyhydric alcohols, glycols such as glycerol and propylene glycol or mixtures thereof being preferred. A mixture or blend of glycerol and propylene glycol is especially preferred, the ratio of the components being selected so that the density of the blend is similar to that of the latent lubricant additive in order to enhance storage time or shelf life by minimising the risk of separation. When the composition is applied to a condom, the ratio of components may also be selected to provide good migration into the rolls of the packaged condom, the volume of the lubricant composition being able to be kept to a minimum while still providing enhanced lubricity in use, once activated by contact with body fluids. The preferred glycerol content of the mixture is between 50% to 90%, and the most preferred is between 65% to 85%. Dispersing the lubricant additive into the carrier lubricant leads to some increase in viscosity and this should be taken into account when selecting the ratio of glycerol to propylene glycol to achieve a specific viscosity. However, a glycerol:propylene glycol ratio of 80:20 by weight has been found to be useful in reducing excessive sedimentation or flotation of the lubricant additive in most formulations.

For a smooth, non-stringing lubricant the preferred lubricant additive is poly(ethylene oxide) of molecular weight 400,000 to 4,000,000. For a lubricant that becomes stringy on contact with body fluids (pituitous) and which has properties similar to natural mucus, a poly(ethylene oxide) with a molecular weight of at least 4,000,000 and up to 5,000,000 or 6,000,000 is preferred. Preferably the poly(ethylene oxide) is of a sufficiently small particle size that it does not feel gritty to the touch. The amount of poly(ethylene oxide) used is preferably in the range 0.5% to 6%, most preferably 1% to 4%, expressed by weight of the overall composition. For poly(ethylene oxide) having a molecular weight of 1 x 10⁶, a concentration of 6% is preferred, especially for application to a condom, whereas a preferred formulation for a personal lubricant for direct application to the penis or the vagina would be 4% or lower, for example 1%, at a molecular weight of 4 x 10⁶. For a higher molecular weight polymer, the amount for application to a condom may be reduced to say 2% or even lower.

Prior to applying the lubricant composition to the condom, there is a tendency for the poly(ethylene oxide) to separate from the carrier lubricant. This effect can be minimised by using a poly(ethylene oxide) with a very small particle size, preferably below 50 micron, and by using a ratio of glycerol to propylene glycol such that the density of the glycol mixture approximately matches the density of the poly(ethylene oxide) particles. Nevertheless, the lubricant composition is preferably stirred vigorously prior to applying it to the condom, to ensure that the poly(ethylene oxide) is fully dispersed. Once the lubricant has been applied to the condom, some separation of the poly(ethylene oxide) may occur but this is not deleterious to the condom, nor to the lubricating properties of the lubricant composition.

The amount of lubricant composition according to the invention as applied to a condom may be between 0.25 and 0.75g, preferably between 0.35 and 0.65g, for example 0.5g. The composition may be injected into the rolled condom either immediately before or at the same time as the condom is packaged, and migration along the entire length of the rolled condom will take place in approximately one week, similar to a silicone fluid lubricant having a viscosity of 350cSt. However, the concentration of latent lubricant additive may be increased to 30 to 35% by weight to form a viscous paste, which may then be applied to one region, for example the closed end region, of the condom, where it will remain localised during storage without substantial migration. In use and following activation by vaginal secretions or by application of saliva to the condom after application to the erect penis, to pre-activate the latent lubricant to enable easier penetration of the penis within the vagina, the lubricating effect between the vaginal wall and the condom is thus optimised.

Optionally, a thickening agent may be added to the composition to increase the viscosity and form a firm gel and to reduce any tendency to settlement and separation. Suitable thickening agents include fumed silica and Carbopol polymers. For use as a personal lubricant for direct topical application, fumed silica (for example Cab-O-Sil M-5P) may be used in a range from 1.5 to 5.0% by weight of the composition, preferably 2.0 to 3.0% by weight, whereas for application to a condom the fumed silica may be used in a range from 0.1 to 1.5% by weight, preferably 0.25% to 0.75% by weight. If a thickening agent is included, the amount of gelled lubricant on the condom can be reduced to up to around 250 mg, for example 100 to 200 mg, and a silicone lubricant should preferably be applied to the condom. In such applications, the ratio of glycerol should be increased relative to propylene glycol, for example within the range 80 to 100%, to inhibit mixing of the lubricant composition with the silicone fluid. By way of example, compositions including a thickening agent may comprise 25 to 35% poly(ethylene oxide) dispersed in a mixture of 80% glycerol and 20% propylene glycol containing from 1 to 5% of a thickening agent to form a firm gel. To facilitate applying the gel to a condom, a volatile solvent, such as ethanol or ispropanol, may be used to dilute the gelled lubricant to an acceptable viscosity for application to the condom. After application to the condom, the solvent is allowed to evaporate before the silicone lubricant is added and the condom is finally sealed in the pack.

Embodiments of the invention will now be described by way of example.

A range of lubricant compositions was produced using the following formulations:

**TABLE 1**

| Glycerol | Propylene Glycol | Poly(ethylene oxide) (PEO) | |
|---|---|---|---|
| (%) | (%) | Mol Wt | (%) |
| 50 | 50 | 600,000 | 2 |
| 35 | 65 | 600,000 | 2 |
| 20 | 80 | 600,000 | 2 |
| 50 | 50 | 1,000,000 | 2 |
| 35 | 65 | 1,000,000 | 2 |
| 20 | 80 | 1,000,000 | 2 |
| 50 | 50 | 4,000,000 | 2 |
| 35 | 65 | 4,000,000 | 2 |
| 20 | 80 | 4,000,000 | 2 |
| 35 | 65 | 4,000,000 | 3 |
| 35 | 65 | 4,000,000 | 4 |
| 65 | 35 | 4,000,000 | 4 |
| 80 | 20 | 4,000,000 | 4 |

In all cases the lubricants became highly lubricious and slippery when rubbed between the fingers with a small amount of water. Lubricity was improved by increasing the PEO content from 2% to 4%. Lubricants containing PEOs with molecular weights of 600,000 and 1 million produced smooth, highly slippery lubricants after activation with water that did not exhibit any stringing. Lubricants containing PEO 4 million were stringy and mucus-like after activation (pituitous behaviour).

It has been found that the quality of lubrication after activation by moisture can be further improved by increasing the PEO content. For example, lubricant formulations containing 5% PEO were very lubricious after activation and lubricant formulations containing 8% were extremely lubricious. The preferred range for a condom lubricant is between 4% and 8%.

For all compositions having a glycerol content of less than 65%, the PEO settled to the bottom of the mixture over a period of 24 hours or so. In the case of formulations containing 65% glycerol, the amount of PEO that settled out within this time was much reduced. There was no settling out of PEO in the case of the formulation containing 80% glycerol.

The Table below summarises the properties of the lubricants:

**TABLE 2**

| PEO Mol Wt | PEO (%) | Properties No Water | Properties With Water |
|---|---|---|---|
| 1,000,000 | 2 | Moderate viscosity | Limited improvement over dry lubricant |
| 1,000,000 | 4 | Moderate viscosity | Good additional lubrication |
| 1,000,000 | 6 | Moderate viscosity | Very good additional lubrication |
| 1,000,000 | 8 | Moderate viscosity | Outstanding additional lubrication |
| 4,000,000 | 2 | Moderate viscosity | Good additional lubrication |
| 4,000,000 | 4 | Moderate viscosity | Very good additional lubrication - slightly pituitous |
| 4,000,000 | 6 | Moderate viscosity | Excellent additional lubrication - moderately pituitous |
| 4,000,000 | 8 | Moderate viscosity | Outstanding additional lubrication - difficult to wash off - highly pituitous |

Particular examples of compositions according to the invention are shown in Table 3, for a personal lubricant (Example 1) and for application to a condom (Example 2) respectively, in each case based on an 80:20 glycerol:monopropylene glycol (MPG) ratio with PEO having a molecular weight of 4,400,000 (commercially available as PEO-18 from RITA Corporation).

**TABLE 3**

| | Ex.1 | Ex. 2 |
|---|---|---|
| Glycerol | 78.4 | 79.2 |
| MPG | 19.6 | 19.8 |
| PEO | 2.0 | 1.0 |
| | 100 (% by weight) | 100 (% by weight) |

The formulation of Example 1 may also contain fumed silica (Cab-O-Sil M-5 or M-5P) at 1-4% by weight, in order to obtain a gel with the correct texture and feel and to prevent separation of the PEO on storage. Example 2 preferably also contains fumed silica at a lower concentration, typically at 0.5% by weight, partly to eliminate any sight sedimentation on extended storage and partly to adjust the viscosity to around 350cSt, similar to that of the silicone fluid normally used as a condom lubricant.

## Claims

1. A personal lubricant composition comprising a mixture of a latent lubricant additive and a carrier lubricant, in which the latent lubricant additive is insoluble in the carrier lubricant, the latent lubricant additive being in particulate or powder form and water-soluble, and comprising a poly (ethylene oxide) polymer, **characterised in that** the carrier lubricant comprises one or more polyhydric alcohols whereby the latent lubricant is capable of activation on contact with body fluids to realise lubricant properties.

2. A composition according to claim 1, in which the carrier lubricant comprises glycerol.

3. A composition according to claim 1, in which the carrier lubricant comprises propylene glycol.

4. A composition according to claim 2, in which the carrier lubricant comprises a mixture or blend of glycerol and propylene glycol.

5. A composition according to claim 4, in which glycerol is present at a concentration of 50 to 90% by weight.

6. A composition according to any preceding claim, in which the polymer is finely-divided and dispersed in the carrier lubricants as a stable suspension.

7. A composition according to any preceding claim, in which the lubricant additive polymer comprises poly (ethylene oxide) of molecular weight 100,000 to 12,000,000.

8. A composition according to claim 7, in which the amount of poly (ethylene oxide) used is in the range 1% to 6% expressed by weight of the overall composition.

9. A composition according to any preceding claim, including a thickening agent.

10. A condom having applied thereto a lubricant composition according to any preceding claim.

11. A condom according to claim 10, in which the amount of lubricant composition applied to the condom is between 0.25 and 0.75g.

12. A condom according to claim 10 or claim 11, in which the concentration of latent lubricant additive is in the range 30 to 35% by weight, the resulting viscous paste being applied to one region of the condom.

13. A condom according to any of claims 10 to 12, in which the poly(ethylene oxide) polymer has a concentration in the range 4 to 8% by weight of the lubricant composition.

14. A condom according to any of claims 10 to 13, in which the composition includes a thickening agent to form a gel, the amount of gelled lubricant on the condom being up to around 250mg and a silicone lubricant being applied to the condom.

15. A condom according to claim 14, in which the ratio of glycerol relative to propylene glycol is within the range 80 to 100%, to inhibit mixing of the lubricant composition with the silicone lubricant.

16. A method of preparation of a condom according to claim 14 or claim 15, in which a volatile solvent is used to dilute the gelled lubricant to a viscosity suitable for application to the condom, the solvent being allowed to evaporate before the silicone lubricant is added and the condom is sealed in a pack.

## Patentansprüche

1. Zusammensetzung für ein Intimgleitmittel, umfassend eine Mischung aus einem latenten Gleitmitteladditiv und einem Trägergleitmittel, wobei das latente Gleitmitteladditiv unlöslich in dem Trägergleitmittel ist, das latente Gleitmitteladditiv in Partikel- oder Pulverform vorliegt und wasserlöslich ist und ein Poly(ethylenoxid)-Polymer umfasst, **dadurch gekennzeichnet, dass** das Trägergleitmittel einen oder mehrere mehrwertige Alkohole umfasst, wodurch das latente Gleitmitteladditiv bei Kontakt mit Körperflüssigkeiten aktivierbar ist, so dass es Gleitmitteleigenschaften gewährt.

2. Zusammensetzung nach Anspruch 1, wobei das Trägergleitmittel Glycerin umfasst.

3. Zusammensetzung nach Anspruch 1, wobei das Trägergleitmittel Propylenglykol umfasst.

4. Zusammensetzung nach Anspruch 2, wobei das Trägergleitmittel eine Mischung oder ein Gemenge aus Glycerin und Propylenglykol umfasst.

5. Zusammensetzung nach Anspruch 4, wobei Glyzerin in einer Konzentration von 50 bis 90 Gewichtsprozent vorhanden ist.

6. Zusammensetzung nach einem der vorherigen Ansprüche, wobei das Polymer fein verteilt und in den Trägergleitmitteln als eine stabile Suspension dispergiert ist.

7. Zusammensetzung nach einem der vorherigen Ansprüche, wobei das Gleitmitteladditivpolymer Poly(ethylenoxid) mit Molekulargewicht 100.000 bis 12.000.000 umfasst.

8. Zusammensetzung nach Anspruch 7, wobei die Menge an verwendetem Poly(ethylenoxid) im Bereich von 1 % bis 6 % liegt, ausgedrückt als Gewicht der Gesamtzusammensetzung.

9. Zusammensetzung nach einem der vorherigen Ansprüche, die ein Verdickungsmittel enthält.

10. Kondom, das darauf appliziert eine Gleitmittelzusammensetzung nach einem der vorherigen Ansprüche aufweist.

11. Kondom nach Anspruch 10, wobei die Menge an Gleitmittelzusammensetzung, die auf dem Kondom appliziert ist, zwischen 0,25 und 0,75 g beträgt.

12. Kondom nach Anspruch 10 oder Anspruch 11, wobei die Konzentration des latenten Gleitmitteladditivs im Bereich von 30 bis 35 Gewichtsprozent liegt, wobei die sich ergebende viskose Paste auf einen Bereich des Kondoms appliziert wird.

13. Kondom nach einem der Ansprüche 10 bis 12, wobei das Poly(ethylenoxid)-Polymer eine Konzentration im Bereich von 4 bis 8 Gewichtsprozent der Gleitmittelzusammensetzung aufweist.

14. Kondom nach einem der Ansprüche 10 bis 13, wobei die Zusammensetzung ein Verdickungsmittel enthält, um ein Gel zu bilden, wobei die Menge an geliertem Gleitmittel auf dem Kondom bis zu etwa 250 mg beträgt und ein SilikonGleitmittel auf das Kondom appliziert wird.

15. Kondom nach Anspruch 14, wobei sich das Verhältnis von Glyzerin relativ zu Propylenglykol innerhalb des Bereichs von 80 bis 100 % befindet, so dass die Mischung der Gleitmittelzusammensetzung mit dem Silikongleitmittel zu verhindert wird.

16. Verfahren zur Herstellung eines Kondoms nach Anspruch 14 oder Anspruch 15, wobei ein flüchtiges Lösemittel verwendet wird, um das gelierte Gleitmittel auf eine Viskosität zu verdünnen, die zur Applikation auf das Kondom geeignet ist, wobei dem Lösemittel ermöglicht wird, zu verdampfen, bevor das Silikongleitmittel hinzugefügt und das Kondom in einer Packung verschlossen wird.

## Revendications

1. Composition de lubrifiant personnel comprenant un mélange d'un additif lubrifiant latent et d'un lubrifiant entraîneur, dans laquelle l'additif lubrifiant latent est insoluble dans le lubrifiant entraîneur, l'additif lubrifiant latent étant sous une forme particulaire ou pulvérulente et hydrosoluble, et comprenant un polymère de poly(oxyde d'éthylène), **caractérisée en ce que** le lubrifiant entraîneur comprend un ou plusieurs alcools polyhydriques grâce à quoi le lubrifiant latent est capable d'activation au contact avec des fluides corporels pour réaliser des propriétés lubrifiantes.

2. Composition selon la revendication 1, dans laquelle le lubrifiant entraîneur comprend du glycérol.

3. Composition selon la revendication 1, dans laquelle le lubrifiant entraîneur comprend du propylène glycol.

4. Composition selon la revendication 2, dans laquelle le lubrifiant entraîneur comprend une mixture ou un mélange de glycérol et de propylène glycol.

5. Composition selon la revendication 4, dans laquelle du glycérol est présent à une concentration de 50 % à 90 % en poids.

6. Composition selon l'une quelconque des revendications précédentes, dans laquelle le polymère est finement divisé et dispersé dans les lubrifiants entraîneurs sous forme d'une suspension stable.

7. Composition selon l'une quelconque des revendications précédentes, dans laquelle le polymère additif lubrifiant comprend du poly(oxyde d'éthylène) de poids moléculaire de 100 000 à 12 000 000.

8. Composition selon la revendication 7, dans laquelle la quantité de poly(oxyde d'éthylène) utilisé est dans le domaine de 1 % à 6 % exprimé en poids de la composition totale.

9. Composition selon l'une quelconque des revendications précédentes, comprenant un agent épaississant.

10. Préservatif ayant une composition lubrifiante appliquée sur celui-ci selon l'une quelconque des revendications précédentes.

11. Préservatif selon la revendication 10, dans lequel la quantité de composition lubrifiante appliquée au préservatif est comprise entre 0,25 g et 0,75 g.

12. Préservatif selon la revendication 10 ou la revendication 11, dans lequel la concentration d'additif lubrifiant latent est dans le domaine de 30 % à 35 % en poids, la pâte visqueuse résultante étant appliquée à une région du préservatif.

13. Préservatif selon l'une quelconque des revendications 10 à 12, dans lequel le polymère poly(oxyde d'éthylène) présente une concentration dans le domaine de 4 % à 8 % en poids de la composition lubrifiante.

14. Préservatif selon l'une quelconque des revendications 10 à 13, dans laquelle la composition comprend un agent épaississant pour former un gel, la quantité de lubrifiant gélifié sur le préservatif allant jusqu'à environ 250 mg et un lubrifiant de silicone est appliqué au préservatif.

15. Préservatif selon la revendication 14, dans lequel le rapport du glycérol par rapport au propylène glycol est dans le domaine de 80 % à 100 %, pour empêcher le mélange de la composition lubrifiante avec le lubrifiant de silicone.

16. Procédé de préparation d'un préservatif selon la revendication 14 ou la revendication 15, dans lequel un solvant volatile est utilisé pour diluer le lubrifiant gélifié à une viscosité appropriée pour l'application sur le préservatif, on laisse s'évaporer le solvant avant que le lubrifiant de silicone soit ajouté et le préservatif est emballé de manière étanche dans un emballage.
